# EUROPEAN PATENT APPLICATION

(11) **EP 4 517 927 A1**
(43) Date of publication of application: **05.03.2025**
(21) Application number: 23819997.0
(22) Date of filing: 08.05.2023
(51) Int. Cl.: H01M 10/54, C22B 3/00, C22B 21/00, C22B 26/12, C12N 13/00, C12N 1/20, C22B 3/18

(54) **METHOD FOR RECOVERING METALS FROM WASTE BATTERY USING MAGNETIC FIELD APPLIED BIOLOGICAL LEACHING PROCESS**

(30) Priority: 08.06.2022 KR 20220069651
(71) Applicant: E-RMB.Inc, Seoul 04763 (KR)
(72) Inventor: YOON, Chong Seung, Seoul 03703 (KR); KIM, Jeon, Seoul 07569 (KR)
(74) Representative: Beck & Rössig European Patent Attorneys
(86) International application number: PCT/KR2023/006194
(87) International publication number: WO 2023/239061

(57) **Abstract**

The present invention relates to a method for recovering metals from waste batteries by using a magnetic field-applied bioleaching process. According to the present invention, by applying a magnetic field to a bioleaching process, it is possible to improve the recovery rate of metals, and to leach valuable metals. In addition, the method is environmentally friendly compared to a dry melting process or an acid leaching process, which has been mainly used for metal recovery, and is practical due to a simple process thereof. Therefore, the method for recovering metals of the present invention may recover metals from wastes in waste resources field such as waste batteries and waste catalysts and turn the metals into resources.

## Description

### BACKGROUND OF THE INVENTION

### 1. Field of the Invention

The invention relates to a method for recovering metals from waste batteries by using a magnetic field-applied bioleaching process, and more particularly, to a method for recovering metals from waste batteries, the method significantly improving the metal recovery efficiency by an eco-friendly process in which a magnetic field application system is combined with a bioleaching process.

### 2. Description of the Related Art

As the demand for portable electronic products increases year by year, the use of secondary batteries is also steadily increasing, and such an increase in demand inevitably generates a large amount of waste batteries.

Waste batteries contain harmful metals such as lead, cadmium, and mercury, and use potassium hydroxide, ammonium chloride, lithium salts, sulfuric acid, an organic solution, or the like as an electrolyte solution, so that the impact of the water batteries on the environment cannot be ignored. In addition, since waste batteries contain valuable metals such as silver, cobalt, nickel, zinc, manganese, and lithium, secondary use of the waste batteries is required to protect the environment and efficiently use finite resources.

The industry of secondary use of waste batteries is attracting attention as companies become more interested in environmental issues, and the market size is expanding as the supply price of secondary battery raw materials rises and competition to attract supply chains intensifies.

Technologies for secondary use of waste batteries are divided into recycling and reuse methods. The recycling method is for extracting expensive metals, mainly from waste batteries for IT devices, and is divided into pre-treatment and post-treatment processes. Elimination of the risk of explosion of waste batteries, magnetic and specific gravity sorting, crushing, and the like are classified as pre-treatment processes, and a dry process, a wet processes, direct recycling, and the like, which actually recover and extract metals, are classified as post-treatment processes.

The reuse method is a method of applying medium-and-large-sized waste batteries mainly from electric vehicles to large batteries such as ESS, and since the capacity and safety of the waste batteries are examined and then modules thereof are disassembled and analyzed to reuse products of a certain grade or higher, there is a limitation in that it is difficult to reuse batteries that do not meet the grade.

In order to improve the recovery rate of waste battery materials through recycling or reuse processes, mobility companies such as Tesla and Volkswagen, secondary battery companies such as Northvolt and CATL, and chemical companies such as LG Chem and BASF are developing technologies, and Tesla is leading the market by introducing a technology that can recover about 92% of battery cell raw materials.

However, except for some companies leading the market, most companies involved in recycling waste batteries have not been able to secure a higher recovery rate than a recovery rate of about 60%. In addition, the dry process, which is mainly used, has a technical limitation in separating metals other than cobalt in a molten state, and the wet process using an acid has a problem of environmental pollution caused by a waste acid, so that there is a demand for the development of a technology for secondary use of waste batteries to replace the above-described processes or improve the same.

As an example of research to solve the above-described limitation and problem, Korean Patent Laid-open Publication No. 10-0796369 discloses a method for recovering valuable metals and recycled plastics from waste batteries, and describes a method for concentrating and recovering each of cobalt and copper, which are valuable metals with high added value, from waste lithium-ion batteries by using a dry melting method, but there is a problem in that melting of the waste lithium-ion batteries requires a considerable amount of energy, and also, costs are wasted due to a complex process involving melting, cooling, and separation steps.

In addition, Korean Patent Laid-open Publication No. 10-2022-0038416 discloses a method for recycling waste batteries through a wet process, and descries that a recycling efficiency of 50% or more can be achieved by using concentrated sulfuric acid, but there is a disadvantage in that the use of a strong acid may cause environmental pollution.

In such a situation, the inventors of the invention have studied a metal recovery method capable of obtaining a high recovery rate for valuable metals in waste batteries through only a simple and eco-friendly process, and as a result, have completed the invention.

### SUMMARY OF THE INVENTION

The purpose of the invention is to provide a method for recovering metals from waste batteries in an eco-friendly and highly efficient manner by applying a magnetic field to a bioleaching process.

In order to achieve the above-described objective, the invention provides a method for recovering a metal from a waste battery, the method including culturing an acidophilic microorganism in a medium to which a magnetic field is applied, adding a waste battery pulverized material to the medium to leach a metal, and recovering the leached metal.

In the invention, the leaching of the metal may be performed in a state in which a magnetic field is applied.

In the invention, the acidophilic microorganism may be one or more among *Acidithiobacillus ferrooxidans, Acidithiobacillus thiooxidans,* and *Acidithiobacillus caldus.*

In the invention, the leached metal may include one or more among cobalt (Co), nickel (Ni), lithium (Li), and aluminum (Al).

In the invention, the culturing may be performed for 3 days to 7 days.

In the invention, the medium may have an initial pH of 1.5 to 3.5.

In the invention, the magnetic field may be direct current (DC) or alternating current (AC).

In the invention, the magnetic field may be direct current, and the magnetic field may have a magnetic flux density of 5 G to 90 G.

In the invention, the magnetic field may be alternating current, and the magnetic field may have a magnetic flux density of 1 G to 10 G.

### ADVANTAGEOUS EFFECTS

The invention applies a magnetic field application system to a bioleaching process of waste batteries, thereby providing a simpler, safer, and more environmentally friendly metal recovery method than a typical dry melting and acid leaching processes.

According to the invention, the growth of microorganisms is improved through the application of a magnetic field, thereby improving the recovery efficiency of metals, which may result in leaching a large amount of metals from waste batteries, and the loss of recyclable resources may be minimized, which is economical.

### BRIEF DESCRIPTION OF THE DRAWINGS

Fig. 1a schematically shows a disk-type magnetic field applying device according to an embodiment of the invention;
Fig. 1b shows an image of culturing microorganisms using a disk-type magnetic field applying device according to an embodiment of the invention;
Fig. 2a schematically shows a cylinder-type magnetic field applying device according to an embodiment of the invention;
Fig. 2b shows an image of culturing microorganisms using a cylinder-type magnetic field applying device according to an embodiment of the invention;
Fig. 3 shows results of measuring the pH according to whether a magnetic field is applied, in an embodiment of the invention;
Fig. 4 shows an image of a flask in which a microorganism is cultured according to whether a magnetic field is applied, in an embodiment of the invention; and
Figs. 5a to 5d show results of measuring the leaching concentration of cobalt (A), lithium (B), nickel (C), and aluminum (D) by using *Acidithiobacillus ferrooxidans* according to a magnetic field type, in an embodiment of the invention.

### DETAILED DESCRIPTION OF THE EXEMPLARY EMBODIMENTS

Unless defined otherwise, all technical and scientific terms used herein have the same meaning as commonly understood by one of ordinary skill in the art to which the invention belongs. In general, the nomenclature used herein is well known and commonly used in the art.

The invention relates to a method for efficiently recovering metals included in waste batteries, and more particularly, to an eco-friendly metal recovery method for improving a metal recovery rate through a simple and safe process of applying a magnetic field during a bioleaching process.

In general, a waste battery includes expensive metals such as cobalt, lithium, and nickel, and the metals are very important target metals in the industry of secondary use of waste batteries.

In order to recover metals from waste batteries, dry or wet processes have generally been used, but the dry process generates a large amount of sulfur gas, and thus, causes many problems in facility operation, and the wet process using an acid uses strong acid and strong alkali solvents during metal leaching, and thus, has the disadvantage of causing very serious environmental pollution.

In order to solve the above-described problems, a bioleaching process, which is a more eco-friendly leaching method, has been proposed, and such a leaching process is a type of a wet process for recovering valuable metals using microorganisms capable of leaching metals.

Specifically, the biological leaching process is a technology which uses a feature in which microorganisms obtain energy necessary for metabolism by combining anions on the surface of the microorganisms with metal cations dissolved in water and metal ions are reduced to metals and precipitated, and when such microorganisms are used, metal ions dissolved in water may be precipitated into solid particles and obtained.

The above-described bioleaching process may solve environmental problems occurring from a dry melting process, and does not require strong acid and strong alkali solvents, and thus is eco-friendly since wastewater may be reduced, and also, compared to other dry and wet processes, has the advantages of low investment and operating costs, easy process control, and no advanced technical knowledge required. However, the bioleaching process has the limitation of having a lower metal recovery rate than other generally used processes.

In the invention, by applying a magnetic field application to a typical bioleaching process, the recovery rate of metals is maximized and even valuable metals included in small amounts in waste batteries, and thus, are not detected or are detected only in small amounts may be recovered with high efficiency.

In this aspect, the method for recovering metals from waste batteries of the invention may be performed by steps of culturing an acidophilic microorganism in a medium to which a magnetic field is applied, adding a waste battery pulverized material to the medium to leach a metal, and recovering the leached metal.

In the invention, as a microorganism to be applied to the bioleaching process, a microorganism capable of leaching metals from various industrial wastes may be used, and an acidophilic microorganism capable of dissolving a metal oxide included in a waste battery in a solution to form a metal salt, and then leaching a metal through a direct or indirect oxidation reaction with the formed metal salt may be used.

Specifically, the above-described microorganism may be selected from acidophilic *Acidithiobacillus* species, and it is preferable to use one or more among *Acidithiobacillus ferrooxidans, Acidithiobacillus thiooxidans,* and *Acidithiobacillus caldus,* wherein *Acidithiobacillus ferrooxidans* is most preferred.

The microorganism may be first cultured in a medium and prepared to be applicable to a metal recovery process. In this case, a medium known in the art which is commonly used for culturing microorganisms may be used, and for example, a medium such as 9K Medium, Medium 271, DSMZ 882, or DSM 71 may be used.

Since microorganisms used for metal leaching require an inorganic compound to grow as chemolitho-autotrophs, it is preferable to include nitrogen, phosphorus, magnesium, and the like in the medium. For example, for optimal growth of the microorganisms, it is preferable to use a medium supplied with ammonia salt, phosphate, magnesium salt, and the like.

It is important to set an initial pH of the medium for growth of an acidophilic microorganism and for metal extraction activity after the growth. The initial pH of the medium may be 1.5 to 3.5, preferably 1.8 to 3.0. The pH of the medium increases to a certain peak according to the growth of the microorganism and then decreases, and metal leaching from a waste battery may be performed at the pH peak.

A stirrer or a mixer for air injection is equipped in the medium, thereby facilitating oxygen supply for the growth of the microorganism. Stirring may be performed at a rate of 100 to 200 rpm, preferably 120 to 180 rpm. If the rate is out of the above-described rate range, an appropriate amount of oxygen for growing the microorganism may be not supplied, so that the growth of the microorganism is degraded, which may result in decreasing the recovery rate of metals.

The microorganism may be cultured at a temperature of 20 °C to 40 °C, preferably 25 °C to 35 °C. The temperature is a factor that has the greatest effect on the metabolism and growth of the microorganism, and when cultured at a temperature out of an appropriate range, cell concentration may be significantly reduced, which may lead to a decrease in the recovery rate of metals.

In the invention, the culturing of the microorganism may be performed under the application of a magnetic field. Since the culturing of the microorganism is promoted due to the application of a magnetic field, it is possible to shorten a period controlled from the initial pH to an appropriate pH range in which the leaching efficiency is maximized, and the metal leaching activity of the microorganism is increased, so that the disadvantage of a bioleaching process, which is a low metal recovery rate, may be resolved. In an embodiment of the invention, it has been confirmed that valuable metals such as Co, Ni, and Li, which are hardly leached or leached at a low yield in a typical bioleaching process, may be recovered at a high concentration.

If a microorganism is cultured under the application of a magnetic field, the growth of the microorganism is promoted, so that even if the microorganism is cultured for a relatively short period of time, it is possible to culture microorganism having an excellent metal extraction activity. Therefore, it is preferable to culture a microorganism for 3 to 7 days. In an embodiment of the invention, it has been confirmed that if a microorganism is cultured in a culture medium to which a magnetic field is applied, the pH of the medium peaks at about 2.5 after only 5 days.

In the invention, a direct current (DC) or alternating current (AC) may be applied to the application of a magnetic field, but in terms of maximizing the leaching efficiency of Co, Ni, and Al, which have been difficult to recover in a typical bioleaching process, the alternating current is preferred. In an embodiment of the invention, it has been confirmed that when an alternating current magnetic field is applied to a medium, the leaching concentrations of Co, Ni, and Al are significantly improved while maintaining the yield of Li without a significant drop compared to when a direct current magnetic field is applied.

At the time of the application of a magnetic field, a magnetic flux density may be 1 to 90 G.

In the invention, if the type of the magnetic field is direct current (DC), the magnetic flux density may be 5 to 90 G, preferably 10 to 80 G, and more preferably 30 to 50 G. In this regard, in an embodiment of the invention, microorganism culturing and metal leaching were performed by changing magnetic flux density conditions to 5, 40, and 90 G when applying a direct current magnetic field, and as a result, it has been confirmed that the leaching efficiency of cobalt, lithium, and nickel are most excellent under the condition of 40 G.

In the invention, if the type of the magnetic field is alternating current (AC), the magnetic flux density may be 1 to 10 G, preferably 2 to 8 G, and more preferably 3 to 7 G. In this regard, in an embodiment of the invention, microorganism culturing and metal leaching were performed by changing magnetic flux density conditions to 1, 5, and 10 G when applying an alternating current magnetic field, and as a result, it has been confirmed that the leaching efficiency of cobalt, lithium, and nickel are most excellent under the condition of 5 G.

The application of the magnetic field may be performed by any method as long as a magnetic field may be applied to a medium, and may be applied using, for example, a disk-type or cylinder-type magnetic field applying device.

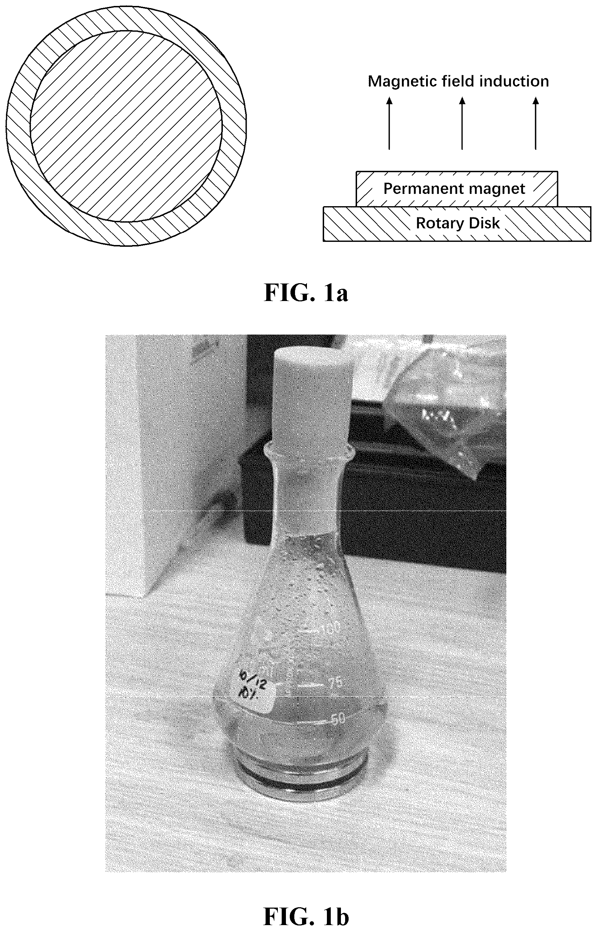

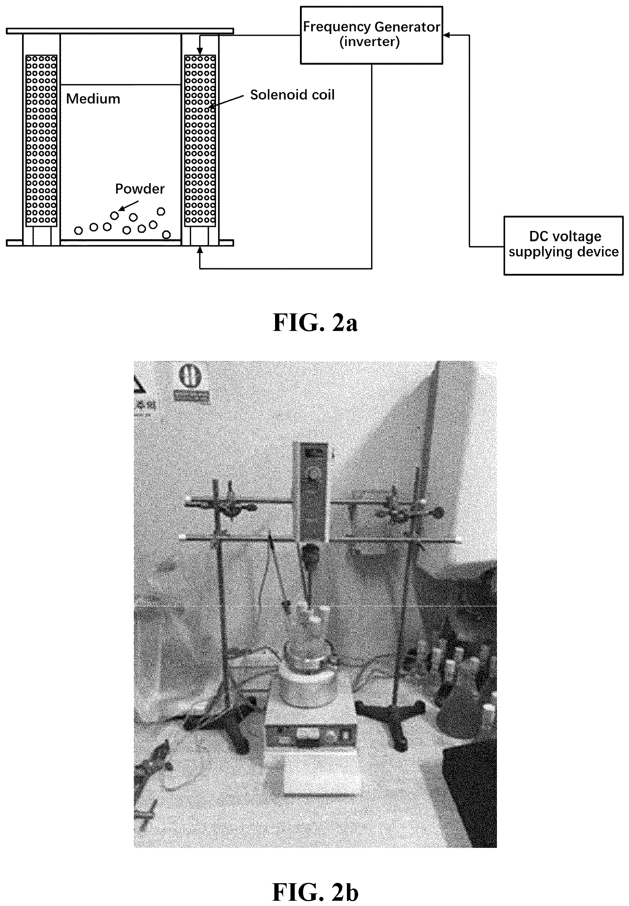

Figs. 1 and 2 show images of exemplary disk-type and cylinder-type magnetic field applying devices and an image in which the magnetic field applying device is applied to microorganism culturing. As illustrated in Fig. 1a, the disk-type magnetic field applying device is configured to install a permanent magnet on a circular rotary disk and dispose a microorganism culturing medium thereon to culture microorganisms, and as illustrated in Fig. 1b, the microorganism culturing medium may be placed on a disk and used.

In addition, the cylinder-type magnetic field applying device is a solenoid device as illustrated in Fig. 2a, and it is possible to culture microorganisms inside the solenoid as in the image of Fig. 1b.

When the acidophilic microorganism is cultured through the above-described steps, a waste battery pulverized material may be added to the medium to leach metals.

The term "waste battery pulverized material" used herein refers to a pulverized material obtained by processing various wastes generated during a manufacturing process of a lithium secondary battery or generated from a waste battery, that is, various types of scraps, jelly rolls, slurries, waste cells, and the like, by a typical pulverization method. To more specifically describe an example of a pretreatment method performed to make solid waste such as collected scraps into a pulverized material form, the solid waste is cut into an appropriate size, primarily classified, and fired to separate an electrode active material and a current collector, and other organic substances and a separator are volatilized. Subsequently, the fired solids are secondarily classified and sorted through specific gravity sorting, magnetic sorting, or the like to obtain a waste battery pulverized material. The waste battery pulverized material thus obtained essentially contains valuable metals such as Co, Ni, and Li, and contains metals such as Al, Fe, and Cu, and carbon, but may have various compositions depending on the type of a waste battery.

After adding the above-described waste battery pulverized material to the medium in which the acidophilic microorganism is cultured, a step of leaching metals may be performed for a predetermined period of time.

The leaching step may be performed at 20 °C to 35 °C, preferably 26 °C to 31°C. As in the culturing step, it is desirable to maintain an optimum temperature during the leaching step so as not to interfere with the metabolism of the microorganism.

In addition, the medium may be stirred at a rate of 100 to 200 rpm for effective leaching, and preferably, may be stirred at a rate of 120 to 180 rpm.

In this case, the longer the leaching time, that is, the reaction time between the microorganism and the waste battery pulverized material, the better the leaching concentration may be, since the increase in the leaching concentration slows down after an initial certain period of time has elapsed, it is preferable to perform the process until it is difficult to expect a significant increase in the concentration. The time for leaching metals with optimal efficiency relative to the reaction time may be 3 days to 30 days, preferably 5 days to 21 days.

In an embodiment of the invention, the metal leaching may also be performed under the application of a magnetic field. The magnetic field may be applied to have the same application conditions as those in the microorganism culturing step, and specifically, may be applied with an alternating current, wherein the magnetic flux density may be 1 to 90 G.

In the invention, the culturing step and the metal leaching step may be simplified to simultaneously perform microorganism culturing and metal leaching by adding a waste battery pulverized material in the microorganism culturing step. In this case, the growth rate of a microorganism may be degraded due to toxicity of the waste battery powder, but there is an advantage in that the process may be simplified.

When a metal is leached from the waste battery pulverized material according to the above-described method, a step of recovering the leached metal may be performed.

The leached metal may be recovered using methods known in the art, such as precipitation, solvent extraction, and electrowinning, and in an embodiment of the invention, the leached metal was recovered through a heat treatment precipitation method in which heat is applied to a leached metal to dry the same.

The method for recovering metals from waste batteries according to the invention has the advantages of being eco-friendly, having a high metal recovery rate, and having a simple process compared to a typical process which has been mainly used, such as a dry melting method and an acid leaching process. In addition, the invention is characterized by adding a magnetic field application system to a bioleaching process, thereby being capable of recovering specific valuable metals which are not easily recovered by the bioleaching process alone, and thus, is suitable to be applied to waste lithium ion batteries containing a relatively large amount of expensive metals.

### Examples

Hereinafter, the invention will be described in more detail with reference to examples. However, these examples show some experimental methods and compositions to illustratively describe the invention, and the scope of the invention is not limited to such examples.

### Experimental Example 1: Analysis of metal leaching concentration according to use of microorganism

*Acidithiobacillus ferrooxidans* and *Acidithiobacillus thiooxidans* were provided by the Korean Collection for Type Cultures (KCTC) and used as a microorganism for leaching metals from waste batteries. The *Acidithiobacillus ferrooxidans* was cultured in an Erlenmeyer flask at 25 °C for 5 days by using 9K medium and adjusting the initial pH to 1.8, and *Acidithiobacillus thiooxidans* was cultured in an Erlenmeyer flask at 25 °C for 7 days by using Medium 271 and adjusting the initial pH to 3.0.

Waste battery powder was added at a concentration of 3 g/100 mL to the flask in which the culturing was completed, and metal leaching was performed for 21 days.

Positive electrode material (Lithium Nickel Cobalt Aluminum Oxide (NCA), LiNiCoAlO₂) powder physically separated from a coin-cell battery was diluted and used as the waste battery powder, which has the composition shown in Table 1 below.

**[Table 1]**

| | Co | Li | Ni | Al |
|---|---|---|---|---|
| | Concentration (ppm) | Concentration (ppm) | Concentration (ppm) | Concentration (ppm) |
| Waste battery powder | 610.392 | 1969.254 | 14769.507 | 67.953 |

An inductively coupled plasma-optical emission spectrometry (ICP-OES) method was performed on the medium in which the metal leaching was completed to analyze the metal leaching concentration, which is shown in Table 2. For comparison, waste battery powder was added to a medium in which a microorganism was not cultured to compare the metal leaching concentration.

**[Table 2]**

| Medium | Microorganism | Co | Li | Ni | Al |
|---|---|---|---|---|---|
| | | Concentration (ppm) | Concentration (ppm) | Concentration (ppm) | Concentration (ppm) |
| 9 K Medium | - | 43.756 | 158.728 | 896.906 | 0.365 |
| 9K Medium | *Acidithiobacillus ferrooxidans* | 46.621 | 723.136 | 1944.719 | 6.964 |
| Medium 271 | - | - | 32.49 | 4.984 | 0.218 |
| Medium 271 | *Acidithiobacillus thiooxidans* | 0.234 | 451.08 | 6.061 | 68.048 |

Referring to Table 2, it can be seen that when a metal was leached using a microorganism, the metal leaching concentration increased compared to when a metal was leached in a medium in which a microorganism was not cultured, and it can be confirmed that the leaching tendency of a metal varies depending on the microorganism and the type of the medium.

Specifically, it can be confirmed that when a metal was leached using *Acidithiobacillus ferrooxidans,* the leaching amount of cobalt was similar, while lithium was leached at a concentration about 4 times higher, nickel about 2 times higher, and aluminum about 20 times higher.

In addition, it can be confirmed that when a metal was leached using *Acidithiobacillus thiooxidans,* cobalt was additionally leached, while lithium was leached at a concentration about 14 times higher, nickel about 1.2 times higher, and aluminum about 300 times higher.

### Experimental Example 2: Analysis of microbial growth rate depending on whether magnetic field is applied

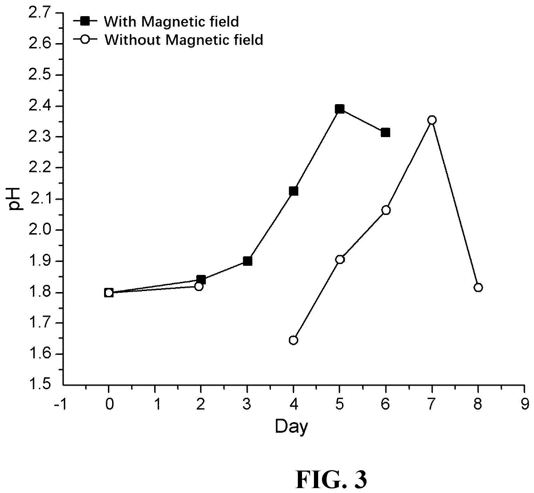

*Acidithiobacillus ferrooxidans* was cultured in the same manner as in Experimental Example 1, but a disk-shaped magnetic field applying device as shown in Fig. 1b was applied to culture the microorganism, and the pH change was compared with that of a medium in which the microorganism was cultured without applying a magnetic field, which is shown in Fig. 3.

Referring to FIG. 3, it can be confirmed that in the case of the medium in which a magnetic field was not applied, the pH was initially 1.8. peaked at 2.36 after 7 days, and then began to decrease, whereas in the case of the medium in which a magnetic field was applied, the pH reached 2.39 in 5 days and then began to decrease, thereby shortening the time taken until the growth of the microorganism was completed by 2 days.

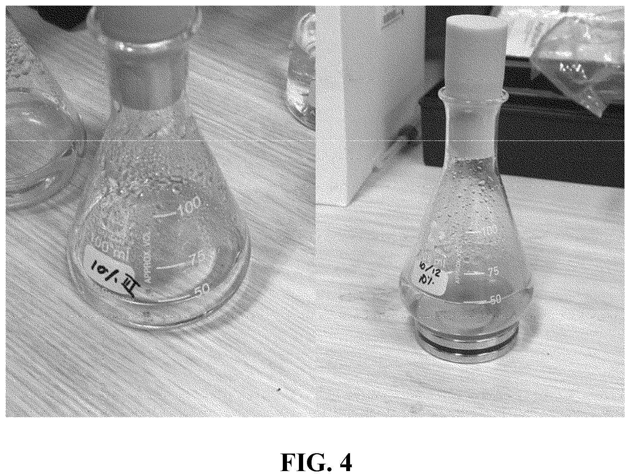

An image of the 4^{th} day of culture in the medium in which a magnetic field was not applied and an image of the 3^{rd} day of culture in the medium in which a magnetic field was applied are compared and shown in Fig. 4.

In Fig. 4, it was confirmed that the medium (right) in which the microorganism was cultured by applying a magnetic field appeared darker than the medium (left) in which a magnetic field was not applied, which was visually recognizable with the naked eye, so that it can be seen that the growth of the microorganism was further promoted in the medium in which a magnetic field was applied.

### Experimental Example 3: Analysis of metal leaching concentration according to magnetic field application method

A microorganism was cultured in the same manner as in Experimental Example 1, but a cylinder-shaped magnetic field applying device (DC: 5G, AC: 3G) as shown in Fig. 2b was applied to culture the microorganism, and diluted waste battery powder was added to the flask in which the culturing was completed to perform metal leaching for 5 days.

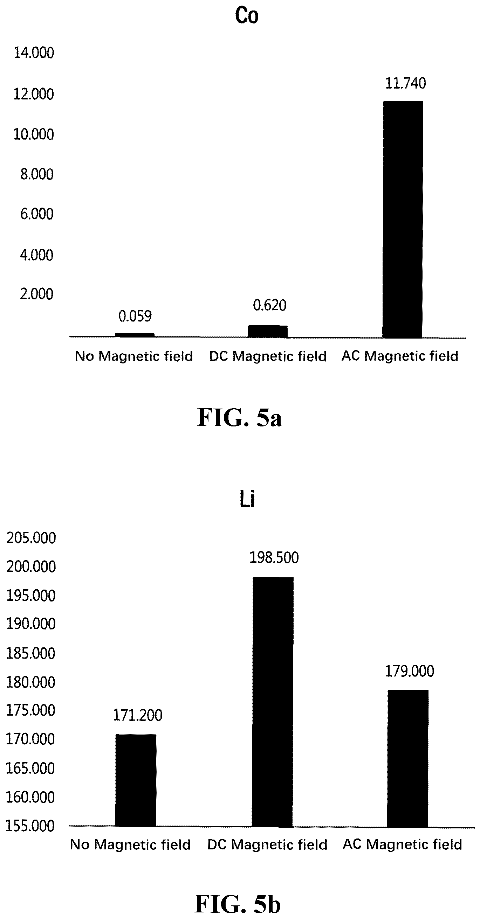

For comparison, the metal leaching concentration was analyzed by leaching a metal from a microorganism cultured without applying a magnetic field and shown in Table 3, of which the leaching concentrations of cobalt, lithium, nickel, and aluminum using *Acidithiobacillus ferrooxidans* were respectively shown in Fig. 5a to Fig. 5d.

**[Table 3]**

| Medium | Microorganism | Magnetic field | Co | Li | Ni | Al |
|---|---|---|---|---|---|---|
| | | | Concentration (ppm) | Concentration (ppm) | Concentration (ppm) | Concentration (ppm) |
| 9 K Medium | *Acidithiobacillus ferrooxidans* | - | 0.059 | 171.2 | 86.12 | 0.096 |
| 9 K Medium | *Acidithiobacillus ferrooxidans* | DC | 0.620 | 198.5 | 179.7 | 0.119 |
| 9 K Medium | *Acidithiobacillus ferrooxidans* | AC | 11.74 | 179 | 349.7 | 0.299 |
| Medium 271 | *Acidithiobacillus thiooxidans* | - | - | 288.800 | 7.492 | - |
| Medium 271 | *Acidithiobacillus thiooxidans* | AC | - | 551.800 | - | - |

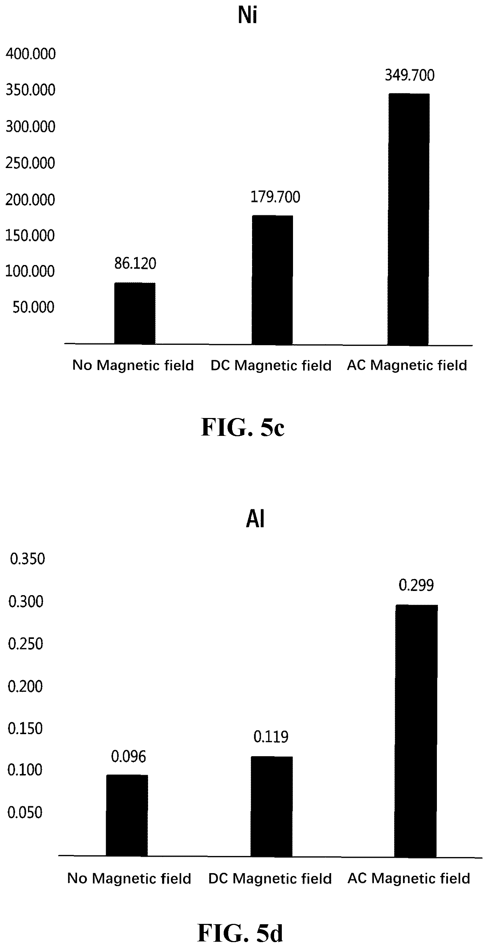

Referring to Fig. 5 and Table 3, the leaching concentrations of Co, Li, Ni, and Al when a direct current magnetic field was applied and when an alternating current magnetic field was applied were measured to be much higher than those when a magnetic field was not applied.

Specifically, when a direct current magnetic field was applied to *Acidithiobacillus ferrooxidans,* the leaching concentration of Co was measured to be about 10 times higher, the leaching concentration of Li was measured to be about 1.15 times higher, the leaching concentration of Ni was measured to be about 2 times higher, and the leaching concentration of Al was measured to be about 1.2 times higher than those when a magnetic field was not applied, when an alternating current magnetic field was applied thereto, the leaching concentration of Co was measured to be about 200 times higher, the leaching concentration of Li was measured to be about 1.05 times higher, the leaching concentration of Ni was measured to be about 4 times higher, and the leaching concentration of Al was measured to be about 3 times higher than those when a magnetic field was not applied.

In addition, when comparing the cases in which the direct current and alternating current magnetic fields were applied, when the alternating current magnetic field was applied, it has been confirmed that the concentrations of Co, Ni, and Al were significantly improved while maintaining the yield of Li without a significant drop.

On the other hand, when an alternating current magnetic field was applied to *Acidithiobacillus thiooxidans,* it has been confirmed that other metals were not detected, but the concentration of lithium increased by about 2 times, and it has been confirmed that *Acidithiobacillus thiooxidans* was not significantly affected by a magnetic field compared to *Acidithiobacillus ferrooxidans.*

### Experimental Example 4: Analysis of metal leaching efficiency according to DC magnetic field flux density condition

Using the method of Experimental Example 3, *Acidithiobacillus ferrooxidans* (*A. ferrooxidans*) was cultured under the condition of applying a DC magnetic field, and waste battery powder was added thereto to perform metal leaching. The same experiment was performed by changing the magnetic flux density to 5, 40, or 90 G, and the leaching efficiency of cobalt, lithium, and nickel for magnetic flux density conditions, oxidation-reduction potential (ORP), and iron ion concentration are shown in Table 4 below.

**[Table 4]**

| Magnetic flux density | Fe³⁺ concentration (g/L) | ORP (mV) | Leaching efficiency (%) | | |
|---|---|---|---|---|---|
| | | | Co | Li | Ni |
| 90G (2.5A) | 3 ± 0.1 | 397 | 37 | 69 | 50 |
| 40G (1A) | 4 ± 0.2 | 387 | 53 | 85 | 54 |
| 5G (0.1A) | 3 ± 0.4 | 382 | 40 | 52 | 40 |
| Normal | 2.5 ± 0.5 | 377 | 32 | 46 | 38 |

From the results of the experiment, it has been confirmed that when the DC magnetic field was applied under the magnetic flux density condition of 5, 40, or 90 G, the leaching efficiency was all improved compared to that of the control group, and particularly, when the magnetic flux density was 40 G, the leaching efficiency of cobalt, lithium, and nickel with respect to the iron ion concentration was respectively 53%, 85%, and 54%, which shows that the leaching efficiency was very excellent.

### Experimental Example 5: Analysis of metal leaching efficiency according to AC magnetic field flux density condition

Using the method of Experimental Example 3, *Acidithiobacillus ferrooxidans (A. ferrooxidans*) was cultured under the condition of applying an AC magnetic field, and waste battery powder was added thereto to perform metal leaching. The same experiment was performed by changing the magnetic flux density to 1, 5, or 10 G, and the leaching efficiency of cobalt, lithium, and nickel for magnetic flux density conditions, oxidation-reduction potential (ORP), and iron ion concentration are shown in Table 5 below.

**[Table 5]**

| Magnetic flux density | Fe³⁺ concentration (g/L) | ORP (mV) | Leaching efficiency (%) | | |
|---|---|---|---|---|---|
| | | | Co | Li | Ni |
| 10G (2.5A) | 5 ± 0.1 | 397 | 35 | 58 | 42 |
| 5G (1A) | 4 ± 0.2 | 387 | 57 | 60 | 63 |
| 1G (0.1A) | 2 ± 0.4 | 382 | 37 | 48 | 52 |
| Normal | 2.5 ± 0.5 | 377 | 32 | 46 | 38 |

From the results of the experiment, it has been confirmed that when the AC magnetic field was applied under the magnetic flux density condition of 1, 5, or 10 G, the leaching efficiency was all improved compared to that of the control group, and particularly, when the magnetic flux density was 5 G, the leaching efficiency of cobalt, lithium, and nickel with respect to the iron ion concentration was respectively 57%, 60%, and 63%, which shows that the leaching efficiency was very excellent.

Although some embodiments of the present invention have been described above, the present invention is not limited to the embodiments described above, and may be performed by modifying and changing the same within a scope that does not depart from the spirit of the present invention, and it should be understood that such modified and changed forms also fall within the technical spirit of the present invention.

## Claims

1. A method for recovering metals from waste batteries, the method comprising:
culturing an acidophilic microorganism in a medium to which a magnetic field is applied;
adding a waste battery pulverized material to the medium to leach a metal; and
recovering the leached metal.

2. The method of Claim 1, wherein the leaching of the metal is performed in a state in which a magnetic field is applied.

3. The method of Claim 1, wherein the acidophilic microorganism is one or more among *Acidithiobacillus ferrooxidans, Acidithiobacillus thiooxidans,* and *Acidithiobacillus caldus.*

4. The method of Claim 1, wherein the metal comprises one or more among cobalt (Co), nickel (Ni), lithium (Li), and aluminum (Al).

5. The method of Claim 1, wherein the culturing is performed for 3 days to 7 days.

6. The method of Claim 1, wherein the medium has an initial pH of 1.5 to 3.5.

7. The method of Claim 1, wherein the magnetic field is direct current (DC) or alternating current (AC).

8. The method of Claim 1, wherein the magnetic field is direct current, and the magnetic field has a magnetic flux density of 5 G to 90 G.

9. The method of Claim 1, wherein the magnetic field is alternating current, and the magnetic field has a magnetic flux density of 1 G to 10 G.
